# EUROPEAN PATENT APPLICATION

(11) **EP 1 935 437 A1**
(43) Date of publication of application: **25.06.2008**
(21) Application number: 06782849.1
(22) Date of filing: 21.08.2006
(51) Int. Cl.: A61L 9/02, A01M 1/20

(54) **EVAPORATION DEVICE AND METHOD OF EVAPORATION**

(30) Priority: 25.08.2005 JP 2005244114
(71) Applicant: Mycoal Co., Ltd., Tochigi-shi, Tochigi 328-0067 (JP)
(72) Inventor: USUI, Kaoru, Tochigi-shi, Tochigi 328-0067 (JP); SAKAMAKI, Yoshikazu, Tochigi-shi, Tochigi 328-0067 (JP); KIMURA, Hisao, Tochigi-shi, Tochigi 328-0067 (JP); KUMAGAI, Yukako, Tochigi-shi, Tochigi 328-0067 (JP)
(74) Representative: Albrecht, Thomas
(86) International application number: PCT/JP2006/316294
(87) International publication number: WO 2007/023755

(57) **Abstract**

The evaporating device 1 comprises a container 10, a heat source 20, which is activated by reacting with water and is placed in the container 10, and an evaporable agent 30 containing an evaporable component, which is put in the water. The heat source 20 comprises a heat-generating composition 23 containing aluminum powder and calcium oxide powder. A predetermined amount of water is added in the container 10 and then the evaporable agent 30 is put in the water directly. The heat-generating composition 23 of the heat source 20 is reacted with the water to cause a heat-generating reaction, producing water vapor and hydrogen gas. The evaporable agent 30 and/or its vapor are evaporated with the water vapor and hydrogen gas. Accordingly, an evaporating device capable of reusing the container and evaporating the evaporable component sufficiently can be provided.

## Description

### Technical Field

The present invention relates to a device and method for evaporating an agent containing an aromatic component or a bactericidal, antibacterial and insecticidal component utilizing water vapor and gas produced by a heat-generating reaction activated by water.

### Background Art

As a device for evaporating an aromatic agent to perfume a room or evaporating an insecticidal agent to exterminate injurious insects, a device utilizing a heat-generating composition activated by adding water has been popularly used (referring to Japanese Patent Publication 2001-294281, for example). The device comprises a container having adjacent compartments separated by walls. In each of the compartments, a solid subject to be heated (evaporable agent), a heat-generating composition which is reacted with water, and water for reacting the heat-generating composition are contained respectively. When the device is used, the wall between the water compartment and the heat-generating composition compartment is broken by a braking means (pin) to communicate the compartments each other, and therefore the water is reacted with the heat-generating composition to cause a heat-generating reaction. The evaporable agent is so positioned at a diffusion path of water vapor and gas produced by the heat-generating reaction as to be evaporated and dispersed into a space.

### Disclosure of the Invention

### Problems to be solved by the Invention

In the aforesaid evaporating device, since consumable supplies such as the evaporable agent, heat-generating composition and water are housed in the container, the container is disposed after use together with the consumable supplies. Accordingly, an amount of the supplies tobe disposed increases and the prices becomes high. And, the container requires water-tightness and strength because it has the compartment containing water.

In view of the problems, the object is to provide an evaporating device and method capable of reusing the container and evaporating the evaporable component sufficiently.

### Means of Solving the Problems

An evaporating device according to the present invention comprises a container; a heat source activated by reacting with water, placed in said container; and an evaporable agent containing an evaporable component to be put in the water, wherein said heat source contains aluminum powder and calcium oxide powder enclosed in a bag, and said evaporable agent is evaporated as follows: a predetermined amount of water is added in said container and then said evaporable agent is put in the water directly; said powders of said heat source are reacted with the water to lead to a following reaction:

CaO+H₂O→Ca(OH)₂+15.2Kcal,

2Al+3Ca (OH)₂→3CaO·Al₂O₃+3H₂+47Kcal,

producing water vapor and hydrogen gas; and said evaporable agent and/or its vapor are dispersed with the water vapor and hydrogen gas.

According to the present invention, the evaporable agent is put in the reacted water directly so that the water mixed with the evaporable agent is boiled to evaporate the evaporable agent by the heat and to diffuse the evaporable agent with the water vapor. Accordingly, it becomes possible to evaporate most of the evaporable agent. Furthermore, since consumable supplies such as the evaporable agent and heat-generating composition are kept separate from the container, the container can be used repeatedly.

In the present invention, it is preferably to place said evaporable agent in said container so as to be surrounded with said heat source.

In this case, since the evaporable agent is placed at the portion to which a sufficient amount of heated water is supplied, the evaporating of the evaporable agent can be activated.

In the present invention, said container is preferably formed with a handle because it becomes easy to carry the container.

In the present invention, the evaporating device preferably comprises means for preventing said heat source from floating in the water because it becomes possible to stabilize the heat-generating reaction of the heat-generating composition.

In the present invention, said heat source may comprise: a bag and a heat-generating composition containing aluminum powder and calcium oxide powder, enclosed in said bag, and said bag is formed by a packing material made of a base material of nonwoven fabric of which one surface is coated with a waterproof layer, said packing material being punched with a pluralities of pinholes and said packing material has a water permeable rate of 45 to 400 milliliter/min/1cm² measured when head of water is 27cm.

According to the present invention, the heat-generating reaction of the heat-generating composition can be controlled by water permeability of the bag. And, by setting a water permeable rate of the bag to 45 to 400 milliliter/min/1cm² measured when head of water is 27cm, the heat-generating reaction can proceed rapidly and a starting of the evaporating can be accelerated.

An evaporating method according to the present invention comprises steps of: placing a heat source, which contains aluminum powder and calcium oxide powder and is activated by reacting with water, in a container and also putting an evaporable agent containing an evaporable component in said container; adding a predetermined amount of water in said container to react said powders of said heat source with the water to lead to a following reaction:

CaO+H₂O→Ca (OH)₂+15.2Kcal,

2Al+3Ca(OH)₂→3CaO·Al₂O₃+3H₂+47Kcal,

producing water vapor and hydrogen gas; and dispersing said evaporable agent and/or its vapor with the water vapor and hydrogen gas.

Examples of the heat-generation composition, nonwoven fabric and evaporable agent for use in the prevent invention are described below.

Examples of the nonwoven fabric include natural fabric such as cotton and wool; regenerated fiber such as viscose (rayon) and cupra; polyamide such as nylon 6, nylon6,6; straight-chain or branched polyesters having 20 or less carbon atoms such as polyethylene terephthalate, polytrimethylen terephthalate, polybutylene terephalate, polylactic acid and polyglycolic acid; polyolefins such as polyethylene and polypropylene; and synthetic fiber such as acrylic. Two or more kinds of those materials may be used together. The nonwoven fabric may be made by a spunlaced method, spunbond method and the like.

Exemplary properties of the nonwoven fabric are followed: basisweight (g/m²); 40~70, thickness (µm) ; 170~460, longitudinal tensile strength (N/5cm); 25~380, transverse tensile strength (N/5cm) ; 13∼165, longitudinal extensibility (%); 80 and below and transverse extensibility (%); 120 and below.

The waterproof layer may be formed by laminating a synthetic-resin film on the nonwoven fabric. Exemplary synthetic-resin films include polyolefin resin such as polyethylene and polypropylene; polyamide resin; polyester resin; polyvinyl chloride resin; polystyrene resin; copolymer polyamide resin; copolymer polyester resin; ethylene-vinyl acetate resin; elastomer; and mixed resin of two or more of those resins. The synthetic-resin film may be a single layer or laminated layer. The synthetic-resin film has a thickness of 0.01 to 0.3mm, preferably 0.02 to 0.1mm

A weight ratio of the aluminum powder to the calcium oxide powder of the heat-generating composition is set to 10:90 - 60:40. Especially, in view of rate of temperature rise and duration of the risen temperature, a weight ratio of the aluminum powder to the calcium oxide powder is preferably set to 35:65 - 50:50.

The aluminum powder preferably has following grain size distribution: ~45µm; 35~60%, 45~63µm; 15~30%, 63~75µm; 5-25% and+75µm; 10-28%. The calcium powder preferably has following grain size distribution: ~75µm; 10~55%, 75~150µm; 25~55% and +150µm; 0~65%.

When an aromatic agent is used for the evaporable agent, examples of the evaporable component (perform material) include natural perfume, isolated perfume and synthetic perfume. And, the component may contain crystalline cellulose, wood flour, dry powder of plant, pulp, regenerated cellulose and fiber, as an impregnant. The impregnant is used for facilitating the handling of the liquid evaporable component by impregnating with the liquid evaporable component. For example, an amount ratio of the liquid perform material to the impregnant is 6:10 - 1:10.

When a bactericidal, antibacterial and insecticidal agent is used for the evaporable agent, examples of the evaporable component (bactericidal, antibacterial and insecticidal substance) include natural antibacterial agent, organic antibacterial agent and inorganic antibacterial agent. And, the component may contain crystalline cellulose, wood flour, dry powder of plant, pulp, regenerated cellulose and fiber, as an impregnant. For example, an amount ratio of the liquid bactericidal, antibacterial and insecticidal component to the impregnant is 8:10.

As described later, the evaporable agent is enclosed in a capsule. As the material of the capsule, cellulose and gelatin may be used. Alternatively, the evaporable agent is not capsulated but solidified into powder or tablet. And, the evaporable agent may be added as a liquid state.

### Effect of the Invention

As described above, according to the present invention, an evaporating device and method capable of sufficiently evaporating an evaporable agent can be provided. And, an evaporating device having a reusable container can be also provided.

### Brief Description of the Drawings

Fig. 1A is a drawing showing a structure of an evaporating device according to the first embodiment of the present invention and Fig.1B is a drawing showing a structure of a heat source contained in the evaporating device of Fig.1A;
Figs.2 are drawings showing the evaporating method according to an embodiment of the present invention, Fig.2A is a plan drawing showing a state in which the heat source and the evaporable agent are set in the container and Fig.2B is a drawing schematically showing a state in which the evaporable agent is being evaporated;
Fig.3 is a drawing showing the evaporating device according to the second embodiment of the present invention;
Fig.4 is a drawing showing the water permeable rate measuring method in the present invention;
Fig.5 is a graph showing a relation between the air permeable rate and the water permeable rate;
Fig.6 is a drawing showing the method for measuring the temperature;
Fig.7 is a graph showing a relation between the water temperature and measuring time of each sample; and
Fig.8 is a graph showing a relation between the environmental temperature and measuring time of each sample.

### Detailed Description of the Preferred Embodiments

Hereinafter, preferred embodiments of the present invention will be precisely described, referring to the drawings.

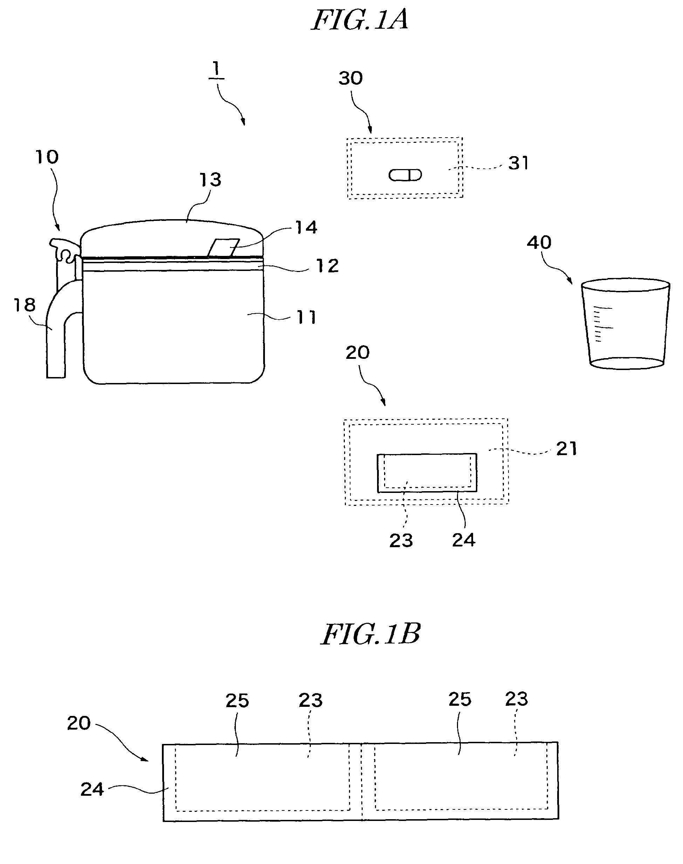

Fig. 1A is a drawing showing a structure of an evaporating device according to the first embodiment of the present invention and Fig.1B is a drawing showing a structure of a heat source contained in the evaporating device of Fig.1A.

The evaporating device 1 comprises a container 10, a heat source 20 placed in the container 10 and an evaporable agent 30 containing an evaporable component. The heat source 20 and the evaporable agent 30 are packed in bags 21 and 31 respectively. And, a measuring cup 40 for measuring water to activate the heat source may be provided.

### (1) Container

The container 10 has a body 11 of a capacity of about 300mililitter, for example. The body 11 is made of polypropylene and has an upper temperature limit of about 120°C. The body 11 is formed with an L-shaped handle 18. The body 11 is closed by an inner lid 12 detachably mounted to the body 11 and an outer lid 13 pivotally mounted to the body 11. The inner lid 12 is formed with a diffusion vent 14 for dispersing water vapor. The inner and outer lids 12 and 13 are made of a material having an upper temperature limit of 100°C or higher such as polypropylene, ABS resin, AS resin, polyethylene, polyvinyl chloride and the like.

### (1) Heat source

The heat source 20 comprises a heat-generating composition 23 comprising a mixed powder of aluminum powder and calcium oxide powder with an amount ratio of the aluminum powder to the calcium oxide powder being 4:6. The heat-generating composition 23 is enclosed in an inner bag 24. For example, the heat-generating composition 23 comprising a mixed powder of aluminum powder (VA-150, manufactured by YAMAISHI METALS Co., Ltd.) of 12g and calcium oxide powder (manufactured by Tagen lime industry) of 18g is used (a total weight is 30g).

In this case, the aluminum powder has the following grain size distribution: ~45µm; 43.52%, 45~63µm; 19.85%, 63~75µm; 18.90% and +75µm; 17.73%. The calcium oxide powder has the following grain size distribution: ~75µm; 11.69%, 75~150µm; 29.27% and +150µm; 59.04%.

The calcium oxide powder consists of the following elements: calcium oxide (measured by an EDTA titration method (NN indicator)); 93% or more, carbon dioxide (measured by a Sutorein method); 2.0% and below and impurities (measured by an EDTA titration method, perchloric acid method, absorption spectroscopy); 3.2% and below. The impurities include silicon dioxide, aluminum oxide, ferric oxide and magnesium oxide.

The heat-generating composition is reacted with water to cause the following heat-generating reaction:

CaO+H₂O→Ca (OH)₂+15.2Kcal,

2Al+3Ca (OH) 2→3CaO·Al₂O₃+3H₂+47Kcal.

By the heat-generating reaction, the water is heated to produce water vapor and gas.

As shown in Fig.1B, the inner bag 24 has a horizontally long shape and has a size of 175mmX35mm. The inner bag 24 is formed with two horizontally long shaped cells 25 in which the heat-generating composition 23 is enclosed. Each cell 25 has a size of 80mmX30mm in this embodiment. 15g of the heat-generating composition 23 is contained in each cell 25. The inner bag 24 may be folded in the length direction at the portion between the cells 25.

As the base material of the inner bag 24, a non water-shedding nonwoven fabric (made by 100% cotton, CO40s/PP40, manufactured by Unitika Ltd.) was used. The nonwoven fabric has the following properties: basis weight (g/m²); 40, thickness (µm); 330, longitudinal tensile strength (N/5cm); 35, transverse tensile strength (N/5cm); 15, longitudinal extensibility (%); 25 and transverse extensibility (%); 75. The nonwoven fabric is made by a spunlaced method in which columnar water flow injects toward fibers at high pressure to entwine the fibers and thus to produce a nonwoven fabric. The spunlaced method allows a production of a highly flexible napless nonwoven fabric having high drape property. A nonwoven fabric produced by the method is used for livelihood materials such as diaper, medical supplies, food supplies and cleaning supplies. Onone surface of the nonwoven fabric, a water-resistant layer (made by polypropylene) was laminated. Or, the water-resistant layer may be made by a heating bonding and the like in exchange for the laminating. Thewater-resistantlayerhadathickness of 40 µm.

Then, water permeability of the bag and a relation between the water permeability and temperature of a heat source will be described.

### <Water permeability>

Sample bags made by base materials which are punched with pinholes in various densities were prepared. And, water permeability (water permeable rate) of each sample was measured.

### (1) Base material

As the base material of the inner bag 24, a non water-shedding nonwoven fabric (made by 100% cotton, CO40s/PP40, manufactured by Unitika Ltd.) was used. The nonwoven fabric has the following properties: basis weight (g/m²) ; 40, thickness (µm) ; 330, longitudinal tensile strength (N/5cm); 35, transverse tensile strength (N/5cm); 15, longitudinal extensibility (%); 25 and transverse extensibility (%); 75. The nonwoven fabric was made by a spunlaced method. On one surface of the nonwoven fabric, a waterproof layer (made by polypropylene) was laminated. The waterproof layer had a thickness of 40µm.

### (2) Pinholes

Each of the prepared base materials was punched with pinholes in various densities using a pinhole opening machine, which comprises a roller on which needles were arranged at intervals of 3. 3mm in the transverse direction and at intervals of 3mm in the longitudinal direction and a base material supporting roller confronting to the former roller. Or, another type of the pinhole opening machine may be used, which is provided with needles capable of being heated and the heated needles are made to contact the laminated film to fuse the film, resulting in opening pinholes. After the base material was supported to the base material supporting roller, each of the rollers rotated in opposite directions. As the result, pinholes having a diameter of 0.1 to 0.4mm were formed on the base material in substantially the uniform density over the almost full area. And, a number of rows of the needles on the roller, or a number of times in which the roller passed on the base material was adjusted to vary the density of the pinholes of the base material. In this embodiment, ten base materials having various pinhole densities were prepared. The pinhole densities varied over a range of 800 to 8000/100cm². If the diameter of the pinhole is larger, the small particulate heat-generating composition may be leaked through the pinhole from the bag, causing unfavorable situation. Accordingly, the pinhole density is preferably 2000 to 8000/100cm², more preferably 3800 to 7100/100cm².

Each of the base materials was cut into a piece having a size of 50mmx50mm to prepare a sample for measuring water permeability.

### (3) Water permeability

There is no official standard showing water permeability of fabric and the like. According to a method for measuring water permeable rate of perforated film, water permeability of each sample was examined by a water permeable rate measuring method, described later.

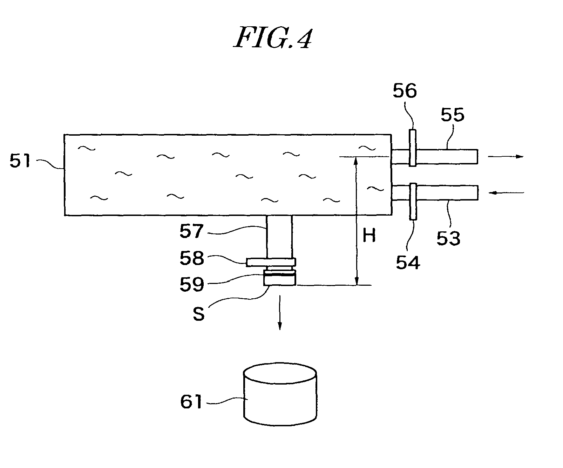

Fig.4 is a drawing showing the water permeable rate measuring method in the present invention.

A stainless-steel measuring tank 51 (inside dimension of 335x535x178mm) was prepared and filled with ion-exchange water of 23±3°C. An inflow pipe 53 from which the ion-exchange water flowed in the tank 51 was formed at the under portion of the side wall of the tank 51 and an overflow pipe 55 was formed at the upper portion of the side wall of the tank 51. The pipes 53 and 55 were openable and closable by cocks 54 and 56, respectively. The ion-exchange water was poured into the tank 51 from the inflow pipe 53 and overflowed through the overflow pipe 55.

An outflow pipe 57 (diameter of 19.05mm) extending downward was formed on the bottom of the tank 51. The outflow pipe 57 was openable and closable by a cock 58. The sample base materials S was temporarily attached to the opening of the outflow pipe 57 by a rubber band 59 with the waterproof surface of the sample S being upside. Then, the periphery of the sample was closely attached to the pipe by a sealing tape to block the opening with the sample S and then further tightly attached by a water impermeable adhesive tape made by polypropylene. A distance H between the opening of the outflow pipe 57 and the overflow port of the overflow pipe 55 was 270±9.5mm (head of water). Under the opening of the outflow pipe 57, a collection vessel 61 was disposed. The collection vessel 61 was set on a measurement apparatus (not shown, GF-3000, manufactured by A&D Co., Ltd.).

The tank 51 was kept the overflow state with the both cocks 54 and 56 opened. When the cock 58 of the outflow pipe 57 was opened, the water was collected by the vessel 61. And, the amount (milliliter) of the collected water was weighed. In this case, after an amount of the permeated water per unit time had got constant (after a variation in amount of the permeated water per 10 seconds was within 5% at least consecutive three times), an amount of the permeated water measured in any one minute during the measurement for one minute or more was defined as a water permeable amount (milliliter). And, a water permeable amount per one minuet per 1cm² of the sample was converted to water permeable rate (milliliter/min/cm²). Aspecificgravity oftheion-exchange water is set to 1.000 (g/cm³).

During the measurement, the flow rate of the ion-exchange water was about 1000milliliter/min. There was little change in the measured amount of permeated water when the flow rate was changed.

Then, a relation between the measured water permeable rate and publicly known air permeability was examined. Because, the measurement of the water permeable rate needs troublesome handling, and, therefore if the water permeable rate is correlated with the air permeability, the measurement of the air permeability can be employed in exchange for the measurement of the water permeable rate.

The air permeability was measured using a gurley type densometer (range; 300ml, timer; s, t<1, a diameter of measuring section; 30mm, manufactured by Toyo Seiki Seisaku-Sho, Ltd., based on JIS P8117). The measured value (sec/300ml) was converted to an air permeable rate (milliliter/min/cm²).

The prepared ten samples having various pinhole densities were examined for water permeability using the aforesaid measuring apparatus and also for air permeability using the gurley type densometer.

Table 1 shows the measured air permeability, air permeable rate converted from the measured air permeability, the measured water permeability and water permeable rate converted from the measured water permeability.

**Table 1**

| Sample No. | air permeability | | water permeability | |
|---|---|---|---|---|
| | measured value | air permeable rate | measured value | water permeable rate |
| | (sec/300ml) | (ml/min./cm²) | (ml/min.) | (ml/min./cm²) |
| 1 | 40.7 | 62.57 | 60.66 | 21.28 |
| 2 | 11.0 | 231.50 | 39.28 | 13.78 |
| 3 | 9.9 | 257.22 | 52.87 | 18.55 |
| 4 | 7.3 | 348.83 | 52.46 | 18.41 |
| 5 | 6.8 | 374.48 | 63.89 | 22.42 |
| 6 | 3.0 | 848.83 | 190.83 | 66.95 |
| 7 | 1.9 | 1340.25 | 301.80 | 105.89 |
| 8 | 1.1 | 2314.98 | 478.23 | 167.79 |
| 9 | 0.7 | 3637.83 | 739.90 | 259.59 |
| 10 | 0.5 | 5092.96 | 1024.70 | 359.51 |

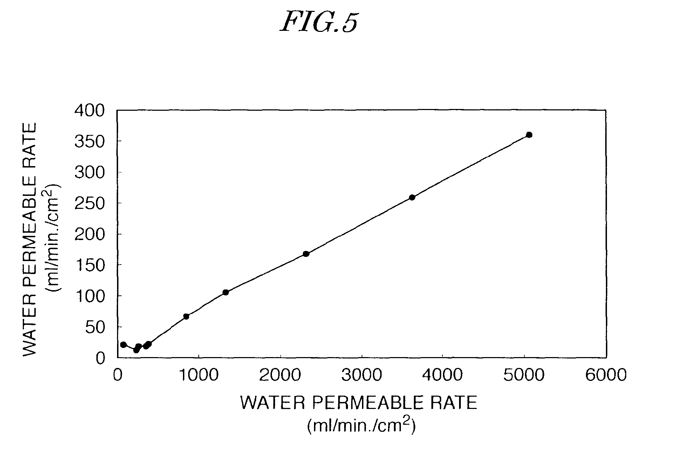

Fig.5 is a graph showing a relation between the air permeable rate and the water permeable rate. The vertical axis indicates the water permeable rate converted from the measured water permeability, and the horizontal axis indicates the air permeable rate converted from the air permeability measured by the gurley type densometer.

As shown in the graph, although the values varies when the both rates are small (when the air permeable rate is less than 400ml/min/cm²), the water permeable rate can be expressed by a direct function of the air permeable rate when the both rate are larger. Accordingly, the graph shows that the water permeable rate is correlated with the air permeable rate. From the graph, a ratio of the water permeable rate to the air permeable rate is substantially equal to 1/13 in a case of the packing material of the present invention.

The following examinations were carried out using the air permeable rate capable of converting to the water permeable rate because the measurement of the water permeable rate is a time-consuming process as described above. So, the water permeable rate was expressed as the air permeable rate divided by 13.

### <Relation between the water permeability of the bag and the temperature of heat source>

A heat source was produced using each of the prepared bags. And, a relation between the temperature of the heat source and the air permeability of the bags was examined.

### (1) Heat-generating composition

As the heat-generating composition, a mixed powder of calcium oxide powder (manufactured by Tagen lime industry) of 30g and aluminum powder (manufactured by YAMAISHI METALS Co., Ltd.) of 20g was used.

### (2) Samples of the bag

The same nonwoven fabric as that used for the measurement of the water permeability was used as the samples of the bag. By varying a number of times in which the roller of the pinhole opening machine passed on the fabric, the samples of nonwoven fabrics in various air permeable rates, described below, were prepared.
Sample 1; 170~250 (milliliter/min/cm²),
Sample 2; 250~400 (milliliter/min/cm²),
Sample 3; 400~600 (milliliter/min/cm²),
Sample 4; 600~1300 (milliliter/min/cm²),
Sample 5; 1300~2000 (milliliter/min/cm²),
Sample 6; 2000~3600 (milliliter/min/cm²),
Sample 7; 3600~4000 (milliliter/min/cm²) and
Sample 8; 4000~5000 (milliliter/min/cm²).

By using the samples, the bag having a receptacle for containing the heat-generating composition was produced. The receptacle had a size of 90mmX150mm.

### (3) Method for measuring temperature

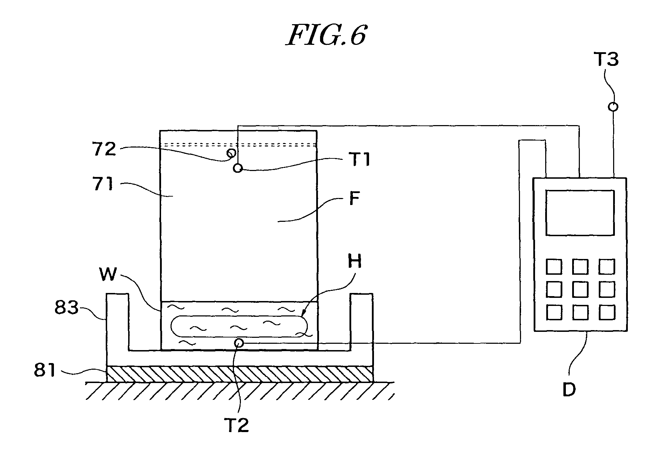

Fig.6 is a drawing showing the method for measuring the temperature.

The heat source H and water W of 130g were put in a heating bag 71 having exhaust vents 72. In this embodiment, the heating bag 71 was openable and closable and had two exhaust vents 72 having a diameter of 5mm. In a temperature-controlled room of which room temperature was kept at 20°C, the heating bag 71 was supported in a stainless-steel container 83 set on a heat insulating material 81. And, for 20 minutes after the heat-generating reaction, a temperature in the heating bag 71 (steam temperature) T1, a temperature T2 of the heated water and an environmental temperature T3 were measured by the measuring apparatus D.

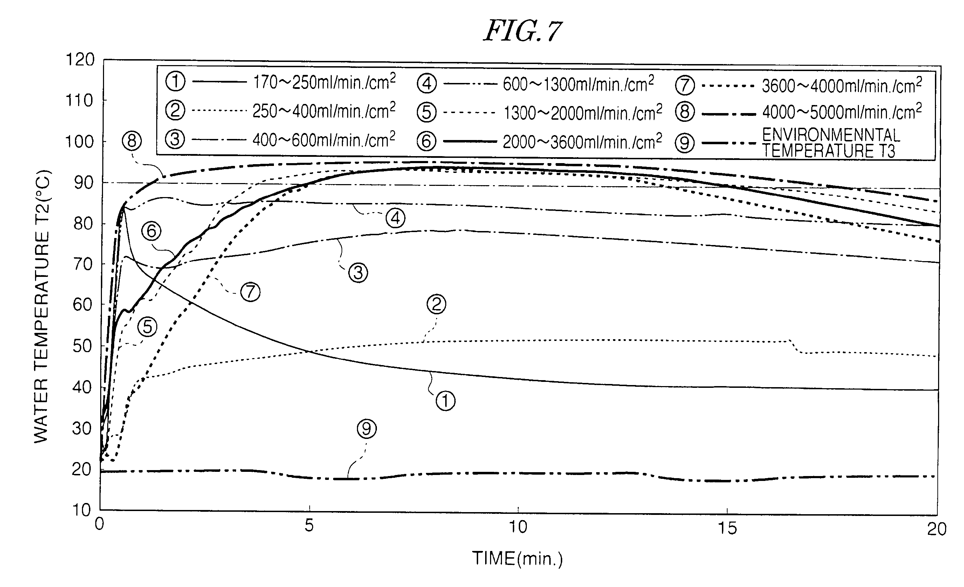

Fig.7 is a graph showing a relation between the water temperature and measuring time of each sample.

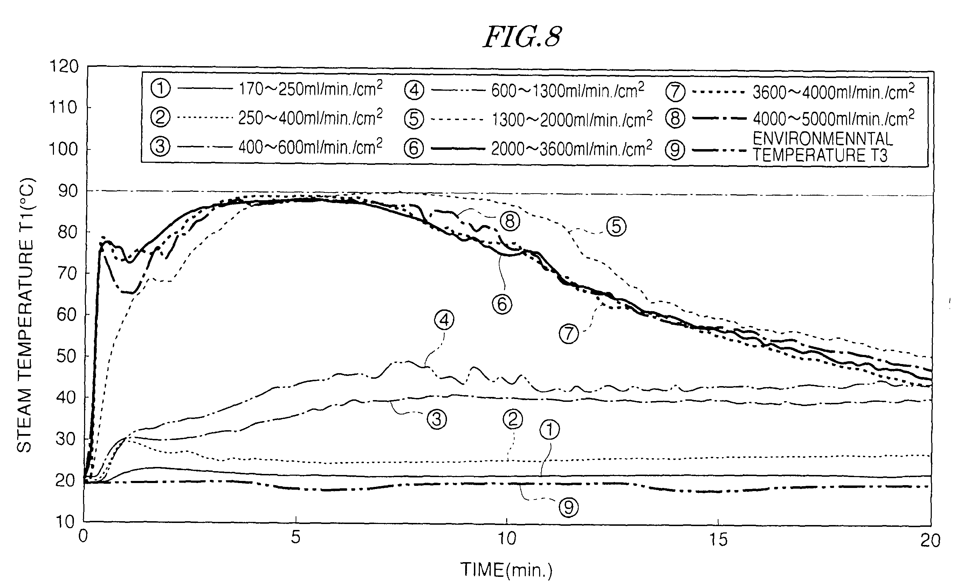

Fig.8 is a graph showing a relation between the environmental temperature and measuring time of each sample.

The horizontal axes indicate a measuring time (minute), and the vertical axes indicate the water temperature T2 (Fig. 7) and environmental temperature T1 (Fig.8).

As shown in Fig. 7, the water temperature T2 of the sample 1 having slow air permeable rate rises rapidly just after the heat-generating reaction; begins to fall down shortly thereafter and falls down to about 40°C after 20 minutes. And, the water temperature T2 of the sample 2 having slow air permeable rate, as well sample 1, rises to about 50°C at a maximum. On the contrary, the water temperature T2 of each of the samples 3, 4 and 5 having middle air permeable rate rises just after the heat-generating reaction, to 70°C or higher after 5 minutes and the risen temperatures is maintained for 20 minutes . The water temperature T2 of each of the samples 6, 7 and 8 having faster air permeable rate rises to 90°C or higher just after the heat-generating reaction, the risen temperatures is maintained for 10 minutes after 5 minutes and then the temperature of 80°C or higher is maintained after 20 minutes.

As shown in Fig.8, the steam temperature (environmental temperature) T1 of each of the samples 1, 2, 3 and 4 having slow air permeable rate does not rise to 50°C or higher. On the contrary, the steam temperature of each of the samples 5, 6, 7 and 8 having fast air permeable rate rapidly rises to 70°C or higher after 2 minutes and the risen temperature of 70°C or higher is maintained for about 10 minutes.

On a basis of Fig.8, how much the air permeable rate is required for the evaporating device was considered. The evaporating device requires a rapid activating of the heat-generating reaction. According to the graph shown in Fig.8, in a case in which the air permeable rate is in the range of 1300 to 5000 milliliter/min/cm² (a measured value by the gurley type densometer is 2.0 to 0.5), a relation between the water permeable rate of the bag and the temperature of heat source was studied.

The sample bags 1 to 4 having air permeable rate of 1300, 1700, 2500 and 5000 milliliter/min/cm² (a measured value by the gurley type densometer is 2.0, 1.5, 1/0 and 0.5) respectively were prepared. A certain amount of heat-generating composition was put in each bag and further water in a weight of 2.5 times of a weight of the heat-generating composition was added to cause a heat-generating reaction. And, whether the samples satisfied the following heating conditions required for the evaporating device was studied.
1) To produce water vapor within 30 seconds.
2) To keep the water vapor blow for 7 minutes or longer.
3) To keep the forceful water vapor blow for 4 minutes after the starting of water vapor production.

Table 2 shows a result whether the samples satisfied the temperature conditions.

**Table 2**

| Sample No. | air permeable rate (ml/min./cm²) | 1) To produce water vapor within 30 seconds | 2) To keep the water vapor blow for 7 minutes or longer | 3) To keep the water vapor blow for 4 minutes after the starting of water vapor production |
|---|---|---|---|---|
| 1 | 1300 | ○ | ○ | ○ |
| 2 | 1700 | ○ | ○ | ○ |
| 3 | 2500 | ○ | ○ | ○ |
| 4 | 5000 | ○ | ○ | ○ |

From these results, the following was obtained:

The air permeable rate of the bag required for the evaporating device is 1300 to 5000 milliliter/min/cm² (water permeable rate is about 45 to 400 milliliter/min/cm²).

According to the result, the bag 24 of the present invention is punched with pluralities pinholes to have a water permeable rate of about 45 to 400 milliliter/min/cm² (in a case in which head of water is 27cm).

### (3) Evaporable agent

As the evaporable agent 30, a capsule filled with an evaporable component (perfume material) was used in this embodiment. The perfume material was a mixture of grapefruit perfume (GRAPE FRUIT 25577, manufactured by KOBAYASHI PERFUMERY CO., LTD.) of 0.35ml and crystalline cellulose (PH-101, ASAHI KASEI CORPORATION) of 0.25g. The capsule is made of cellulose.

Next, an evaporating method will be described.

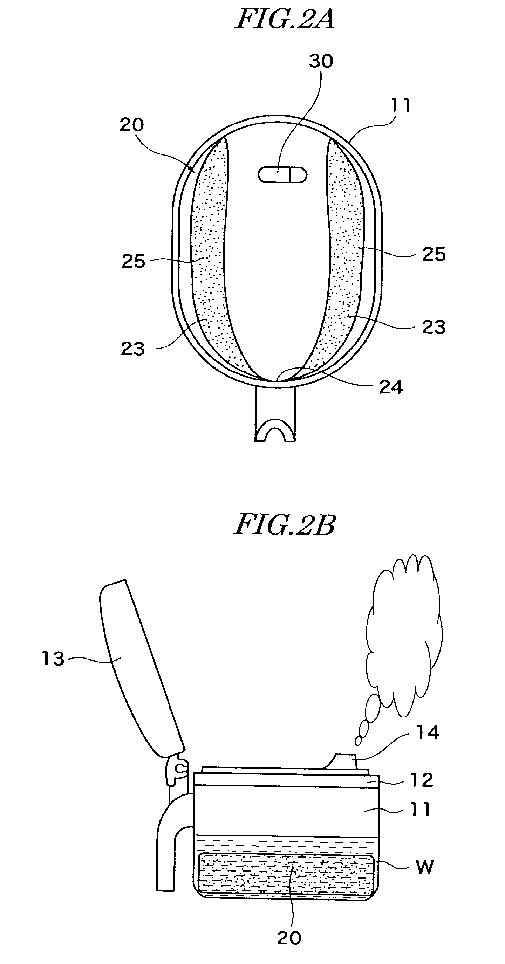

Figs.2 are drawings showing the evaporating method according to an embodiment of the present invention, Fig.2A is a plan drawing showing a state in which the heat source and the evaporable agent are set in the container and Fig.2B is a drawing schematically showing a state in which the evaporable agent is being evaporated.

First, the heat source 20 is taken out of the bag 21 and placed in the body 11 of the container 10. As shown in Fig.2A, the heat source 20 is doubled into a V-shape viewed from above and placed such that each cell 25 stands along the inner wall of the body 11. And, the evaporable agent 30 is taken out of the bag 31 and placed between the cells 25 of the V-shape folded heat source 20. Water of 80 milliliter is weighed using the measuring cup 40 (or separately prepared measuring cup) and then added in the container 10. Immediately thereafter, the inner lid 12 is closed while the outer lid 13 kept opened (as shown in Fig.2B).

Then, the heat-generating composition 23 of the heat source 20 is reacted with the water W to lead to the aforesaid heat-generating reaction. The heat-generating reaction heats the water W and breaks the capsule of the evaporable agent 30, and the perfume material in the capsules begins to be mixed with the water. On proceeding of the heat-generating reaction further, the water with which the perfume material is mixed is boiled and hydrogen is produced. The water vapor and the hydrogen gas are dispersed through the diffusion vent 14 of the inner lid 12.

By evaporating the water with which the perfume material is mixed, the perfume material is dispersed directly. And, since almost all of the water is evaporated, most of the perfume material can be dispersed. Furthermore, since the inner bag 24 of the heat source 20 has the aforesaid water permeability, the reaction of the heat-generating composition with the water proceeds rapidly and the generated heat can be diffused rapidly. Accordingly, it becomes possible to cause the heat-generating reaction in a few moments and therefore to shorten a period before the evaporating.

After the completion of the evaporating, the container 10 can be reused after discarding the heat source 20 and the like. Accordingly, when the evaporable agent 30 and the heat source 20 are sold individually apart from the container, the container 10 can be used repeatedly. And, in a case of aromatic agent, by preparing various types of the evaporable components, it becomes possible to select desirable perfume.

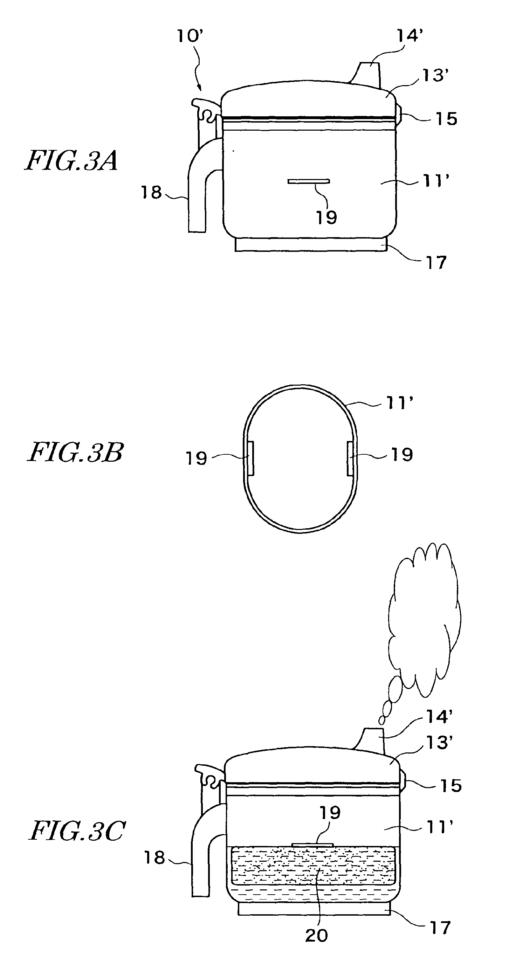

Fig.3 is a drawing showing the evaporating device according to the second embodiment of the present invention.

The evaporating device has the same structure as that of Fig.2 except for the shape of the container in the following.

The container 10' of the embodiment does not have an inner lid; has an outer lid 13' formed with a diffusion vent 14'. And, a fastener 15 for detachably attaching the upper lid 13' to the body 11' is provided. And, the body 11 is formed with a pedestal 17 at the bottom. The pedestal 17 prevents the heat produced by the heat-generating reaction from transmitting to the floor and the like on which the container 10' is set.

The body 11' is further provided with protrusions 19 extending inwardly and horizontally at the inner wall. The protrusions 19 hold down the heat source 20 to prevent from floating in the water, causing a stabilized heat-generating reaction.

In the present invention, amount of each of the evaporable agent, heat source and water varies according to a size of the room into which the evaporable agent is evaporated.

In the present invention, the heat-generating composition may contain calcium hydroxide powder in exchange for calcium oxide powder or both calcium hydroxide powder and calcium oxide powder. An amount ratio of each component may be described below: aluminum powder: calcium hydroxide powder: calcium oxide powder= 30∼60 : 1∼50 : 0∼69, preferably 40~60 : 10~40 : 10~40 and more preferably 40~50 : 15~40 : 20~40.

## Claims

1. An evaporating device comprising a container; a heat source activated by reacting with water, placed in said container; and an evaporable agent containing an evaporable component to be put in the water,
wherein said heat source contains aluminum powder and calcium oxide powder enclosed in a bag, and
said evaporable agent is evaporated as follows:
a predetermined amount of water is added in said container and then said evaporable agent is put in the water directly;
said powders of said heat source are reacted with the water to lead to a following reaction:
CaO+H₂O→Ca(OH)₂+15.2Kcal,
2Al+3Ca (OH)₂→3CaO·Al₂O₃+3H₂+47Kcal,
producing water vapor and hydrogen gas; and
said evaporable agent and/or its vapor are dispersed with the water vapor and hydrogen gas.

2. An evaporating device according to claim 1,
wherein said evaporable agent is so placed in said container as to be surrounded with said heat source.

3. An evaporating device according to claim 1 or 2 further comprising means for preventing said heat source from floating in the water.

4. An evaporating device according to any one of claims 1, 2 or 3,
wherein said container is formed with a handle.

5. An evaporating device according to any one of claims 1 to 4,
wherein said heat source comprises:
a bag and
a heat-generating composition containing aluminum powder and calcium oxide powder, enclosed in said bag, and
said bag is formed by a packing material made of a base material of nonwoven fabric of which one surface is coated with a waterproof layer, said packing material being punched with a pluralities of pinholes and
said packing material has a water permeable rate of 45 to 400 milliliter/min/lcm² measured when head of water is 27cm.

6. An evaporating method comprising steps of:
placing a heat source, which contains aluminum powder and calcium oxide powder and is activated by reacting with water, in a container and also putting an evaporable agent containing an evaporable component in said container;
adding a predetermined amount of water in said container to react said powders of said heat source with the water to lead to a following reaction:
CaO+H₂O→Ca(OH)₂+15.2Kcal,
2Al+3Ca (OH)₂→3CaO·Al₂O₃+3H₂+47Kcal,
producing water vapor and hydrogen gas; and
dispersing said evaporable agent and/or its vapor with the water vapor and hydrogen gas.

7. An evaporating device comprising a container; a heat source activated by reacting with water, placed in said container; and an evaporable agent containing an evaporable component to be put in the water,
wherein said heat source contains aluminum powder and calcium hydroxide powder enclosed in a bag, and
said evaporable agent is evaporated as follows:
a predetermined amount of water is added in said container and then said evaporable agent is put in the water directly,
said powders of said heat source is reacted with the water to lead to a following reaction:
2Al+3Ca (OH) 2→3CaO·Al₂O₃+3H₂+47Kcal,
producing water vapor and hydrogen gas; and
said evaporable agent and/or its vapor are dispersed with the water vapor and hydrogen gas.

8. An evaporating method comprising steps of:
placing a heat source, which contains aluminum powder and calcium hydroxide powder and is activated by reacting with water, in a container and also putting an evaporable agent containing an evaporable component in said container;
adding a predetermined amount of water in said container to react said powders of said heat source with the water to lead to a following reaction:
2Al+3Ca(OH)₂→3CaO·Al₂O₃+3H₂+47Kcal,
producing water vapor and hydrogen gas; and
dispersing said evaporable agent and/or its vapor with the water vapor and hydrogen gas.
